# EUROPÄISCHE PATENTSCHRIFT

(11) **EP 3 305 345 B1**
(45) Veröffentlichungstag und Bekanntmachung des Hinweises auf die Patenterteilung: **02.10.2019**
(21) Anmeldenummer: 17195541.2
(22) Anmeldetag: 09.10.2017
(51) Int. Cl.: A61M 1/36

(54) **VORRICHTUNG ZUR REZIRKULATIONSMESSUNG**
DEVICE FOR RECIRCULATION MEASUREMENT
DISPOSITIF DE MESURE DE LA RECIRCULATION

(30) Priorität: 10.10.2016 DE 102016119259
(43) Veröffentlichungstag der Anmeldung: 11.04.2018
(73) Patentinhaber: B. Braun Avitum AG, 34212 Melsungen (DE)
(72) Erfinder: JANIK, Waldemar, 34212 Melsungen (DE); KRAUSE, Silvie, 34212 Melsungen (DE)
(74) Vertreter: Winter, Brandl, Fürniss, Hübner, Röss, Kaiser, Polte - Partnerschaft mbB

(56) Entgegenhaltungen:
- EP-A1- 1 970 080
- WO-A1-2009/065611
- WO-A2-2017/140424
- DE-A1-102013 103 222
- DE-C1- 19 528 907
- US-A- 5 588 959

## Beschreibung

Die Erfindung betrifft eine Vorrichtung zur quasi-kontinuierlichen Rezirkulationsmessung mittels Thermodilution, und bezieht sich insbesondere auf eine Vorrichtung zur Rezirkulationsmessung mittels Thermodilution bei extrakorporaler Blutbehandlung, die eine zumindest quasi-kontinuierliche Erfassung einer Rezirkulation in einem Blutschlauchsystem eines extrakorporalen Blutkreislaufs bei extrakorporaler Blutbehandlung erlauben.

Bei bekannten Verfahren zur extrakorporalen Blutbehandlung oder auch Blutreinigung, wie beispielsweise Hämodialyse, Hämofiltration und Hämodiafiltration, wird das Blut eines Patienten durch einen extrakorporalen Kreislauf geleitet. Als Zugang zum Blutgefäßsystem wird dem Patienten in der Regel ein sogenannter Shunt gelegt. Ein Shunt ist in diesem Fall eine arteriovenöse Verbindung im Unterarm, die auch mithilfe eines Implantats umgesetzt werden kann. Für eine Dialysebehandlung wird der Shunt in der Regel mit zwei Kanülen punktiert. Über die eine Kanüle wird das Blut aus dem Patienten zur Reinigung zum Dialysator geleitet. Über die zweite Kanüle wird das gereinigte Blut zum Patienten zurückgeführt. Übersteigt der Blutfluss im extrakorporalen Kreislauf den Blutfluss im Shunt des Patienten, führt dies zu einer Rezirkulation, bei der ein Teil des bereits gereinigten Blutes nochmals aus dem Shunt zum Dialysator geführt wird. Ursachen dafür können beispielsweise nicht optimal gelegte Kanülen oder Stenosen im Shunt sein.

Das Auftreten einer Rezirkulation kann daher als Prädiktor für Zugangsversagen dienen, genauer gesagt als ein später Prädiktor bzw. eine späte Vorhersagegrundlage. Da (Shunt-) Rezirkulationen in der Regel nachteilig mit einer Effizienzminderung der Dialysebehandlung einhergehen, kann zwar kurzfristig auf eine rezirkulationsfreie Dialyse abgestellt werden. Im Wesentlichen aber kann ein Auftreten einer Rezirkulation zugunsten einer besseren Diagnose des Shunts und gegebenenfalls schnellstmögliche Einleitung einer Revision herangezogen werden, da Rezirkulationsbestimmungen über einen längeren Zeitraum Auskunft über den Zustand des Shunts liefern können.

Bekannte Maßnahmen bei Rezirkulationsbestimmungen beinhalten eine Erwärmung einer Dialysierflüssigkeit, beispielsweise einer einen Dialysator durchfließenden Dialysierflüssigkeit , ein Messen eines Temperaturbolus bzw. eines Leitfähigkeitsbolus am Dialysierflüssigkeitsausgang eines Dialysators nach Änderung eines physikalischen oder chemischen Parameters am Dialysierflüssigkeitseingang, oder Vorrichtungen, die Temperatursensoren in sowohl einem arteriellen Kreislaufsegment als auch in einem venösen Kreislaufsegment erfordern. Die Größe der bei den bekannten Maßnahmen beteiligten Volumen, wie beispielsweise das der Dialysierflüssigkeit, führt jedoch zu langsamen Änderungen einer jeweils betrachteten Größe und in der Folge zu trägen Systemreaktionen, die Messungen nur in großen Zeitabständen erlauben.

Diese bekannten Maßnahmen können somit keine Messwerte in schneller oder kurzer zeitlicher Folge liefern und sind daher für eine kontinuierliche oder quasi-kontinuierliche Messung ungeeignet. Bislang sind mit den bekannten Maßnahmen keine kontinuierlichen oder zumindest quasi-kontinuierlichen Messungen oder die Bestimmung eines optimalen Blutflusses auf Grundlage kontinuierlich oder quasi-kontinuierlich durchgeführter Rezirkulationsbestimmungsmessungen möglich.

Die Druckschrift US 5,588,959 A offenbart eine Vorrichtung und ein Verfahren für eine Rezirkulationsmessung mittels Temperatur. Dabei wird das Blut über den venösen Schlauchabschnitt gekühlt und die Temperatur des Bluts kann im arteriellen Schlauchabschnitt gemessen werden.

Auch die Druckschrift WO 2009/065611 A1 offenbart eine Vorrichtung zur extrakorporalen Blutbehandlung, bei der mittels Temperatur eine Rezirkulation des Blutes bestimmt werden kann, indem die Temperaturänderung in der Dialysierflüssigkeit erzeugt und über die Dialysatormembran auf das Blut übertragen wird.

Die Druckschrift DE 195 28 907 C1 offenbart ebenfalls eine Vorrichtung zur Rezirkulationsmessung, bei der ein Temperaturbolus im Dialysierflüssigkeitszweig erzeugt wird.

Die Druckschrift DE 10 2013 103 222 A1 offenbart eine Vorrichtung zur Rezirkulationsmessung mittels eines Temperaturbolus im venösen Blutzweig.

Der Erfindung liegt daher als eine Aufgabe zugrunde, eine Vorrichtung für eine einfach und zyklisch in kurzer zeitlicher Abfolge wiederholt durchführbare Rezirkulationsmessung bereitzustellen.

Darüber hinaus soll die Erfindung das Auffinden eines optimalen Blutflusses, bei dem gerade noch keine Rezirkulation auftritt, ermöglichen, wobei dieser optimale Blutfluss auch während einer Therapie ebenfalls quasi-kontinuierlich bestimmbar und einstellbar sein soll.

Erfindungsgemäß wird diese Aufgabe durch eine Vorrichtung mit den Merkmalen des Anspruchs 1 gelöst. Vorteilhafte Weiterbildungen der Erfindung sind Gegenstand der beigefügten Unteransprüche.

In Übereinstimmung mit einer erfinderischen Idee zielt die Erfindung darauf ab, eine Vorrichtung für eine einfach durchzuführende Rezirkulationsmessung zur Verfügung zu stellen, die insbesondere in kurzer zeitlicher Folge wiederholbare, d.h. kontinuierliche und zumindest quasi-kontinuierliche, Rezirkulationsmessungen leistet, wodurch schnelle Reaktionsmöglichkeiten auf Änderung eines Zustands eines Patienten, beispielsweise aufgrund einer Kreislaufbelastung während einer Behandlung, möglich werden. Darüber hinaus kann mit der Vorrichtung und einer geschickten verfahrenstechnischen Umsetzung automatisch der optimale Blutfluss, bei dem gerade noch keine Rezirkulation auftritt, gefunden werden. Dieser Blutfluss kann dann während der Therapie ebenfalls quasi-kontinuierlich bestimmt und eingestellt werden.

Durch zyklische Änderung einer relevanten physikalischen Größe, beispielsweise zyklisches Ändern (Erhöhen/Senken) der Bluttemperatur im venösen Teil des Blutschlauchsegments, und Messung der physikalischen Größe, beispielsweise der Temperatur im arteriellen Segment, kann zum einen die Zugangsrezirkulation bestimmt werden, und kann zum anderen der optimale Blutfluss bestimmt und eingestellt werden, bei dem keine Rezirkulation auftritt. Die Änderung der physikalischen Größe, beispielsweise die Temperaturänderung, kann beispielsweise mithilfe (mindestens) eines Peltier-Elements erfolgen. Erfolgt die zyklische Änderung in kurzer zeitlicher Abfolge, resultiert dies in einer kontinuierlichen und zumindest quasi-kontinuierlichen Messung.

In vorteilhafter Weise sind erfindungsgemäß keine Änderungen sonstiger Einstellparameter, wie z.B. Dialysierflüssigkeits- und Blutfluss, notwendig, wird aufgrund reduzierter Trägheit durch reduzierte Totzeiten eine einfache und schnelle Rezirkulationsmessung möglich, sind Messvorgänge zugunsten einer kontinuierlichen Messung wiederholbar, sind keine Infusionslösungen notwendig, kann ein optimaler Blutfluss automatisch ermittelt werden und kann eine regelungstechnische Anpassung des Blutflusses automatisch erfolgen, und sind ein Überwachen bzw. Monitoring des optimalen Blutflusses und damit ein Shuntflussmonitoring bzw. ein Shuntmonitoring möglich.

Ferner weist eine Rezirkulation bei fortgeschrittener Behandlungszeit in der Regel auf Kreislaufinstabilitäten hin, sodass eine Detektion als Triggerereignis für ein biologisch rückkoppelndes System (Biofeedbacksystem) nutzbar ist, mit welchem dann beispielsweise eine Blutdruckmessung durchführbar und/oder eine Ultrafiltrationsrate anpassbar wird, oder die Zusammensetzung der Dialysierflüssigkeit anpassbar wird.

Im Einzelnen wird die Aufgabe gelöst durch eine Vorrichtung zur Rezirkulationsmessung mittels Thermodilution bei extrakorporaler Blutbehandlung, beinhaltend eine Temperaturänderungseinrichtung, die dazu angeordnet ist, für eine zumindest quasi-kontinuierliche Rezirkulationsmessung Blut in einem venösen Schlauchsegment des Blutschlauchsystems zyklisch zu erwärmen oder abzukühlen; und eine Erfassungseinrichtung, die dazu konfiguriert ist, eine aus der Erwärmung oder Abkühlung des Bluts resultierende Änderung einer physikalischen Größe in einem arteriellen Schlauchsegment des Blutschlauchsystems zu messen, wobei die Erfassungseinrichtung dazu angeordnet ist, in einem rezirkulationsfreien Fall, in dem bei der extrakorporalen Blutbehandlung keine Rezirkulation von venösem Blut in arterielles Blut auftritt, eine erste physikalische Größe zu erfassen, und in einem rezirkulationsbehafteten Fall, in dem bei der extrakorporalen Blutbehandlung eine Rezirkulation von venösem Blut in arterielles Blut auftritt, eine durch das Kühlen oder Erwärmen des Bluts in dem venösen Schlauchsegment durch die Temperaturänderungseinrichtung geänderte physikalische Größe als eine zweite physikalische Größe in dem Blutschlauchsystem zu erfassen. Dabei ist in einer solchen Vorrichtung eine Pumpe dazu angeordnet, Blut in einem Blutschlauchsystem eines extrakorporalen Blutkreislaufs zu pumpen und ist die Pumpe in Abhängigkeit von einer an der Erfassungseinrichtung erfassten Größenänderung dazu ansteuerbar, einen optimalen Blutfluss für eine Dialysebehandlung einzustellen, wobei die Vorrichtung dazu angeordnet ist, ein Eintreten einer Rezirkulation bei Erfassen einer ersten Größenänderung an der Erfassungseinrichtung zu bestimmen und ein Verschwinden der Rezirkulation bei Erfassen einer zweiten Größenänderung an der Erfassungseinrichtung zu bestimmen, einen ersten Blutfluss bei der ersten Größenänderung und einen zweiten Blutfluss bei der zweiten Größenänderung mathematisch zu einem resultierenden Blutfluss zu kombinieren, und die Pumpe dazu anzusteuern, den resultierenden Blutfluss als einen optimalen Blutfluss einzustellen.

Der bevorzugte Temperaturbereich, in dem das Blut gekühlt und/oder erwärmt werden kann bzw. der für die erfindungsgemäße Rezirkulationsmessung geeignet ist, liegt zwischen (einschließlich) 30°C und (einschließlich) 41°C.

Bevorzugt ist in einer solchen Vorrichtung die Temperaturänderungseinrichtung stromab eines Blutausgangs eines Dialysators angeordnet, und/oder ist eine Temperaturerfassungseinrichtung vorgesehen und dazu angeordnet, die Temperatur des Bluts an dem oder in dem venösen Schlauchsegment zu erfassen.

Bevorzugt ist dabei die Temperaturänderungseinrichtung ein Peltier-Element.

Bevorzugt ist die physikalische Größe, die sich mit der Temperaturänderung durch die Temperaturänderungseinrichtung einhergehend ändert, eine einer arteriellen Bluttemperatur zugeordnete Temperatur des Bluts, ein Druck oder eine Kraft.

Bevorzugt ist die Vorrichtung dazu angeordnet, durch das zyklische Erwärmen und Abkühlen des Bluts in dem venösen Schlauchabschnitt einen Temperatursignalverlauf an zumindest einer Temperaturerfassungseinrichtung zu erzeugen, wobei Amplituden des Temperatursignalverlaufs und/oder Flächen unter dem Temperatursignalverlauf zur quasi-kontinuierlichen Bestimmung einer Rezirkulation auswertbar sind.

Bevorzugt ist bei der Vorrichtung eine Rezirkulation durch eine quantitative Maßzahl und/oder eine qualitative Größe bezüglich medizinischer Relevanz der Rezirkulation bestimmbar, wobei die qualitative Größe anhand eines prozentualen Schwellenwerts "Rezirkulation gleich oder größer als X Prozent" mit X als einer zahlenmäßigen Prozentangabe ausgebbar ist.

Die Erfindung wird nachstehend anhand bevorzugter Ausführungsbeispiele unter Bezugnahme auf die Zeichnung näher beschrieben.

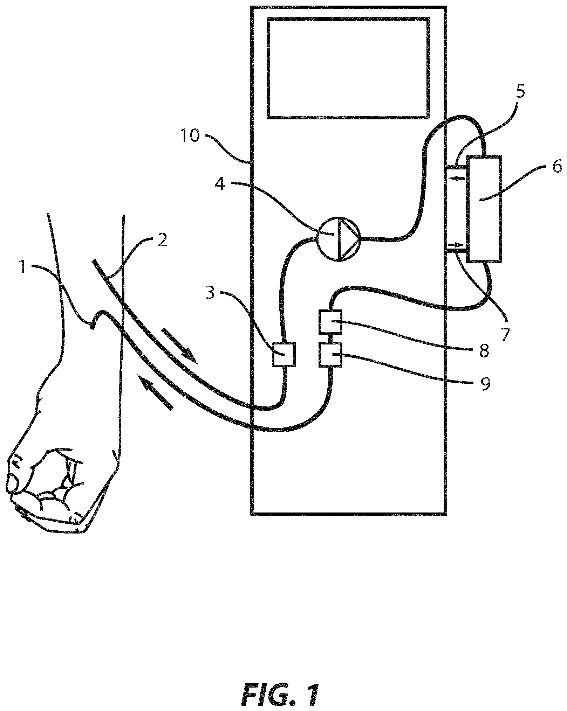

Fig. 1 zeigt eine schematische Konfiguration der Vorrichtung zur Rezirkulationsmessung mittels Thermodilution bei extrakorporaler Blutbehandlung gemäß einem Ausführungsbeispiel, bei dem als relevante physikalische Größe oder relevanter Parameter beispielhaft die Temperatur herangezogen wird. Die Erfindung ist jedoch nicht auf die Temperatur als relevante physikalische Größe oder relevanter Parameter beschränkt. Anwendungsspezifisch sind zahlreiche physikalische Größen und Parameter möglich, solange deren zyklische Änderung in zeitlich kurzer Abfolge eine zumindest quasi-kontinuierliche Messung zur Ermittlung einer auftretenden Rezirkulation erlaubt, wie beispielsweise ein Druck oder eine Kraft.

Im Einzelnen zeigt Fig. 1 in schematischer Andeutung einen Patienten, dem an einem arteriellen Zugang 2 mithilfe einer Pumpe 4, in diesem Ausführungsbeispiel einer Blutpumpe in beispielsweise einem Dialysegerät 10, über ein Blutschlauchsystem Blut entnommen wird. Zwischen dem arteriellen Zugang 2 am Patienten und der Pumpe 4 wird an einem arteriellen Schlauchabschnitt des Blutschlauchsystems mit einer Erfassungseinrichtung 3, die beispielsweise ein zur Temperaturerfassung oder Temperaturmessung geeigneter Sensor oder Messfühler sein kann, eine Temperatur gemessen, die der arteriellen Bluttemperatur zugeordnet werden kann. Das entnommene Blut wird zu dessen Reinigung durch einen Dialysator 6 geleitet. Stromab eines Blutausgangs des Dialysators 6 ist eine bzw. eine erste Temperaturänderungseinrichtung 8 angeordnet, die in dem vorliegenden Ausführungsbeispiel beispielsweise ein Wärme-/Kühlelement nach Art eines Peltier-Elements sein kann. Peltier-Elemente bieten den Vorteil, dass mit ihnen durch Umpolung der Versorgungsspannung sowohl geheizt als auch gekühlt werden kann. Eine Beschränkung auf Peltier-Elemente als Temperaturänderungseinrichtung 8 besteht jedoch nicht. Die Temperaturänderungseinrichtung 8 erwärmt oder kühlt das Blut im venösen Schlauchsegment des Blutschlauchsystems stromab des Dialysators 6. Eine weitere bzw. zweite Temperaturerfassungseinrichtung 9, die ebenfalls ein zur Temperaturerfassung oder Temperaturmessung geeigneter Sensor oder Messfühler sein und als eine Sicherheitsvorrichtung vorgesehen sein kann, um ein zu starkes Abkühlen oder Erwärmen durch die Temperaturänderungseinrichtung 8 schnellstmöglich zu erkennen, kann in dem venösen Schlauchsegment des Blutschlauchsystems angeordnet sein und die Temperatur am oder im venösen Schlauchsegment erfassen.

Zur Ermittlung oder Bestimmung einer Rezirkulation ändert die Vorrichtung die Temperatur des venösen Bluts mithilfe der Temperaturänderungseinrichtung 8. Rezirkulationsbehaftet, d.h. bei Auftreten einer Rezirkulation strömt ein Teil des venösen Bluts zusammen mit dem arteriellen Blut erneut durch das arterielle Schlauchsegment, wodurch aufgrund der geänderten Temperatur des venösen Blutanteils eine Temperaturänderung an der Erfassungsvorrichtung 3 erfassbar wird. Rezirkulationsfrei, d.h. wenn keine Rezirkulation vorliegt, bleibt die Temperatur an der Erfassungseinrichtung 3 unverändert. Durch zyklisches Erwärmen und Abkühlen vermittels der Temperaturänderungseinrichtung 8 kann auf diese Weise quasi-kontinuierlich Rezirkulation bestimmt werden. Zur Auswertung können beispielsweise die Amplituden von an der Erfassungseinrichtung 3 erzeugten Temperatursignalverläufen oder Flächen unter solchen Kurven genutzt werden. Die Rezirkulation kann sowohl als quantitative Maßzahl als auch qualitativ ausgegeben werden. Qualitativ kann in diesem Ausführungsbeispiel bedeuten, dass anhand eines prozentualen Werts bezogen auf beispielsweise Rezirkulationsfreiheit angezeigt wird, ob eine medizinisch relevante Rezirkulation (beispielsweise Rezirkulation > 15%) vorliegt oder nicht. Es versteht sich, dass der prozentuale Wert oder ein Schwellenwert zu dessen Festlegung nicht auf den vorgenannten konkreten Wert beschränkt ist.

In einer Modifikation können die vorstehend beschriebene Vorrichtung und ein entsprechendes Verfahren zur Ermittlung bzw. Bestimmung und/oder Einstellung eines optimalen Blutflusses für eine Dialysebehandlung herangezogen werden.

Dazu kann beispielsweise beim Anlegen des Patienten zu Beginn einer Dialysebehandlung, bei dem der (anfängliche) Blutfluss standardmäßig geringer ist als der Blutfluss während der restlichen Behandlungsdauer, die arterielle Temperatur an der Erfassungseinrichtung 3 gemessen werden. Mit der Temperaturänderungseinrichtung 8 wird zum Patienten über einen Zugang 1 zurückfließendes venöses Blut sodann um eine vorbestimmte Temperaturdifferenz abgekühlt oder erwärmt, und der von der Pumpe 4 erzeugte Blutfluss daraufhin allmählich erhöht. Wenn an der Erfassungseinrichtung 3 eine Temperaturänderung erfasst wird, wird ein Vorliegen einer Rezirkulation bestimmt. In diesem Fall übersteigt der Blutfluss in dem Blutschlauchsystem den Shuntfluss, d.h. den Blutfluss durch den Shunt, im Arm des Patienten und ist somit zu hoch gewählt.

Aufgrund hinreichend kurzer Totzeiten kann der Blutfluss zum Zeitpunkt des Erfassens des Auftretens der Rezirkulation als ein optimaler Blutfluss betrachtet werden. Ein noch genauerer Wert kann erhalten werden, wenn Totzeiten zahlenmäßig bekannt oder zumindest abschätzbar sind und dieser als optimal betrachtete Blutfluss unter Berücksichtigung der Totzeiten um einen bestimmten Wert reduziert wird.

Alternativ kann der Blutfluss nach Erfassen des Auftretens der Rezirkulation solange langsam verringert werden, bis keine weitere Rezirkulation bestimmt wird, d.h. bis die Rezirkulation verschwindet. In dieser Alternative kann der Blutfluss bei Verschwinden der Rezirkulation als der optimale Blutfluss betrachtet werden.

Alternativ oder zusätzlich ist es möglich, den Blutfluss bei einem Erfassen des Auftretens einer Rezirkulation und den Blutfluss bei einem Verschwinden der Rezirkulation zu mitteln, d.h. einen Mittelwert aus entsprechenden Blutflusswerten zu bilden, oder anderweitig geeignet mathematisch zu einem resultierenden Blutfluss zu kombinieren. In diesem Fall entspricht der resultierende Blutfluss dem optimalen Blutfluss.

Darüber hinaus können die Ermittlung und/oder die Einstellung des optimalen Blutflusses automatisch oder manuell erfolgen, wobei in diesem Ausführungsbeispiel unter manuell zu verstehen ist, dass ein Anwender den durch die Pumpe 4 erzeugten Blutfluss manuell bis zum Erfassen des Auftretens einer Rezirkulation erhöht und anschließend gegebenenfalls manuell senkt.

Es versteht sich, dass die Ermittlung des optimalen Blutflusses nicht auf den Beginn einer Dialysebehandlung beschränkt ist. Der optimale Blutfluss kann auch während einer Dialysebehandlung ermittelt werden, beispielsweise nach einer Aufforderung durch den Anwender oder selbsttätig bzw. automatisch, wenn bzw. während die Temperaturänderungseinrichtung 8 das Blut in dem venösen Schlauchabschnitt zyklisch wärmt und kühlt. Des Weiteren ist es auch möglich, den optimalen Blutfluss quasi-kontinuierlich zu bestimmen. Dadurch kann der Blutfluss mithilfe der Pumpe 4 nachgeführt werden.

In einer weiteren Modifikation erlauben die Vorrichtung und das Verfahren gemäß dem Ausführungsbeispiel eine kurzfristige, mittelfristige und/oder langfristige Trendanalyse des optimalen Blutflusses. Hierbei wird für jede Therapie eine charakteristische Wertekombination ermittelt. Dies kann beispielsweise der Median aller während der Behandlung ermittelten Blutflüsse in Kombination mit der Spanne aller Blutflüsse sein.

Auch ein mittlerer Blutflusswert einschließlich der Standardabweichung oder andere adäquate Kombinationen der deskriptiven Statistik sind darstellbar. Die charakteristischen Wertekombinationen können auf einer Patientenkarte, einem Datenmanagementsystem oder direkt auf/in dem Dialysegerät 10 gespeichert werden. Die charakteristischen Wertekombinationen können über die Zeit aufgetragen werden. Die Ableitung der Daten nach der Zeit erlaubt einen Rückschluss auf Veränderungen am Shuntgefäß, da die Ableitung dann negativ wird, wenn der optimale Blutfluss und damit auch der Shuntfluss über die Zeit sinken. Für kurzfristige Trendanalysen werden vorzugsweise zumindest zwei oder optimalerweise drei Behandlungen herangezogen, für mittelfristige Analysen zumindest drei bis neun Behandlungen und für langfristige zumindest zehn. Auf diese Weise kann die Vorrichtung gemäß dem Ausführungsbeispiel aufweisende Dialysemaschine 10 anhand des ermittelten Trends Warnungen ausgeben, wenn eine negative Ableitung auf eine Verschlechterung des (nicht gezeigten) Shunts hindeutet, sodass medizinisches Personal erforderlichenfalls weitere diagnostische Maßnahmen ergreifen kann.

Wie vorstehend beschrieben wurde, ist in einer Vorrichtung zur Rezirkulationsmessung mittels Thermodilution bei extrakorporaler Blutbehandlung eine Temperaturänderungseinrichtung 8, beispielsweise ein Peltier-Element, dazu angeordnet, für eine zumindest quasi-kontinuierliche Rezirkulationsmessung Blut in einem venösen Schlauchsegment des Blutschlauchsystems zyklisch zu erwärmen und/oder abzukühlen, und ist eine Erfassungseinrichtung 3, die beispielsweise ein Temperatursensor, ein Drucksensor oder ein Kraftsensor bzw. -aufnehmer sein kann, dazu konfiguriert, in einem arteriellen Schlauchsegment des Blutschlauchsystems eine physikalische Größe, beispielsweise eine einer arteriellen Bluttemperatur zugeordnete Temperatur des Bluts, zu messen. In einem Fall, in dem bei der extrakorporalen Blutbehandlung keine Rezirkulation von venösem Blut in arterielles Blut auftritt, erfasst die die physikalische Größe erfassende Erfassungseinrichtung 3 eine erste physikalische Größe mit Bezug zu in diesem Fall arteriellem Blut, wobei die erste physikalische Größe zwischen einzelnen Messungen unverändert bleibt. In einem rezirkulationsbehafteten Fall, in dem bei der extrakorporalen Blutbehandlung eine Rezirkulation von Blut aus dem venösen Schlauchabschnitt in arterielles Blut auftritt und das bezüglich der einhergehend mit der Temperaturänderung durch die Temperaturänderungseinrichtung 8 veränderten physikalischen Größe veränderte Blut aus dem venösen Schlauchabschnitt dabei einen Größenänderungseintrag oder einen Größenänderungsaustrag in das bzw. aus dem arterielle(n) Blut verursacht, erfasst die Erfassungseinrichtung 3 eine durch das Kühlen oder Erwärmen des Bluts in dem venösen Schlauchsegment durch die Temperaturänderungseinrichtung 8 geänderte physikalische Größe als eine zweite physikalische Größe des Bluts in dem Blutschlauchsystem.

Für eine Blutzirkulation in dem Blutschlauchsystem sorgt eine Pumpe 4, die als Blutpumpe dazu angeordnet ist, Blut in einem Blutschlauchsystem eines extrakorporalen Blutkreislaufs zu pumpen.

Die Temperaturänderungseinrichtung 8 kann stromab eines Blutausgangs eines Dialysators 6 angeordnet sein. Ferner kann, etwa zu Steuerungs- und/oder Erfassungszwecken, eine Temperaturerfassungseinrichtung 9 im Bereich des venösen Schlauchsegments vorgesehen und dazu angeordnet sein, die Temperatur des Bluts an dem oder in dem venösen Schlauchsegment zu erfassen.

Die Temperaturänderungseinrichtung 8 ist bevorzugt ein Peltier-Element, das durch Umpolen seiner Versorgungsspannung zyklisch im Wechsel zwischen einem Wärmeerzeugungsbetrieb und einem Kühlbetrieb umschaltbar ist und somit dazu betreibbar ist, das Blut in dem venösen Schlauchabschnitt zyklisch zu erwärmen und/oder abzukühlen. Dadurch ist zumindest eine quasi-kontinuierliche Rezirkulationsmessung mit kurzen Totzeiten, das heißt bei rascher Zyklusfolge schneller Ansprech- oder Antwortzeit, erzielbar.

Während eines zyklischen Erwärmens und Abkühlens des Bluts in dem venösen Schlauchabschnitt kann dazu gemäß dem Ausführungsbeispiel ein Temperatursignalverlauf an der in diesem Fall eine Temperatur erfassenden Erfassungseinrichtung 3 erzeugt werden, dessen Amplituden und/oder Flächen unter der Verlaufskurve zur quasi-kontinuierlichen Bestimmung einer Rezirkulation auswertbar sind.

Im Rahmen einer Auswertung ist eine Rezirkulation durch beispielsweise eine quantitative Maßzahl und/oder eine qualitative Größe bezüglich medizinischer Relevanz der Rezirkulation bestimmbar. Die qualitative Größe kann dabei als ein prozentualer Wert oder Schwellenwert "Rezirkulation gleich oder größer als X Prozent" bezogen auf den rezirkulationsfreien Fall mit X als einer zahlenmäßigen Prozentangabe ausgegeben werden.

Ferner kann die Pumpe 4 in Abhängigkeit von einer an der Erfassungseinrichtung 3 erfassten Änderung der physikalischen Größe dazu ansteuerbar sein, einen optimalen Blutfluss für eine Dialysebehandlung einzustellen. Genauer kann die Pumpe 4 dazu ansteuerbar sein, bei Erfassen einer Größenänderung an der Erfassungseinrichtung 3, aufgrund der das Auftreten einer Rezirkulation bestimmt wird, den Blutfluss zu verringern, bis eine Größenänderung an der Erfassungseinrichtung 3 erfasst wird, aufgrund der das Verschwinden der Rezirkulation bestimmt wird, wobei der Blutfluss, bei dem die Rezirkulation verschwindet, als ein optimaler Blutfluss eingestellt wird.

Alternativ kann ein Auftreten oder Eintreten einer Rezirkulation bei Erfassen einer ersten Änderung einer physikalischen Größe an der Erfassungseinrichtung 3 bestimmt werden, und kann ein Verschwinden der Rezirkulation bei Erfassen einer zweiten Änderung einer physikalischen Größe an der Erfassungseinrichtung 3 bestimmt werden, kann ein erster Blutfluss bei der ersten Größenänderung und ein zweiter Blutfluss bei der zweiten Größenänderung mathematisch zu einem resultierenden Blutfluss kombiniert werden, und kann auf dieser Grundlage die Pumpe 4 dazu angesteuert werden, den resultierenden Blutfluss als einen optimalen Blutfluss einzustellen.

Die Erfindung wurde vorstehend anhand eines bevorzugten Ausführungsbeispiels beschrieben. Es versteht sich, dass Einzelheiten des beschriebenen bevorzugten Ausführungsbeispiels die Erfindung als solche nicht beschränken und sich für den Fachmann naheliegend verschiedenartige Änderungen, Modifikationen und/oder Äquivalente ergeben können, die als solche allesamt im Rahmen der durch die beigefügten Ansprüche definierten Schutzbereich der Erfindung liegen.

Insbesondere versteht sich, dass sich dem Fachmann für den Fall einer anderen physikalischen Größe als der Temperatur zu den entsprechend temperaturbezogenen Komponenten gemäß dem Ausführungsbeispiel ohne Weiteres äquivalente oder entsprechende Komponenten ergeben, die sich zur Erfassung und Verarbeitung der anderen physikalischen Größe eignen und dazu konfigurierbar sind.

## Patentansprüche

1. Vorrichtung zur Rezirkulationsmessung mittels Thermodilution bei extrakorporaler Blutbehandlung, mit:
einer Temperaturänderungseinrichtung (8), die dazu angeordnet ist, für eine zumindest quasi-kontinuierliche Rezirkulationsmessung Blut in einem venösen Schlauchsegment des Blutschlauchsystems zu erwärmen und/oder abzukühlen; und
einer Erfassungseinrichtung (3), die dazu konfiguriert ist, eine aus der Erwärmung und/oder Abkühlung des Bluts resultierende Änderung einer physikalischen Größe in einem arteriellen Schlauchsegment des Blutschlauchsystems zu messen, wobei
die Erfassungseinrichtung (3) dazu angeordnet ist, in einem rezirkulationsfreien Fall, in dem bei der extrakorporalen Blutbehandlung keine Rezirkulation von venösem Blut in arterielles Blut auftritt, eine erste physikalische Größe zu erfassen, und in einem rezirkulationsbehafteten Fall, in dem bei der extrakorporalen Blutbehandlung eine Rezirkulation von venösem Blut in arterielles Blut auftritt, eine durch das Kühlen und/oder Erwärmen des Bluts in dem venösen Schlauchsegment durch die Temperaturänderungseinrichtung (8) geänderte physikalische Größe als eine zweite physikalische Größe in dem Blutschlauchsystem zu erfassen;
einer Pumpe (4), die dazu angeordnet ist, Blut in einem Blutschlauchsystem eines extrakorporalen Blutkreislaufs zu pumpen, wobei die Pumpe (4) in Abhängigkeit von einer an der Erfassungseinrichtung (3) erfassten Größenänderung dazu ansteuerbar ist, einen optimalen Blutfluss für eine Dialysebehandlung einzustellen, **dadurch gekennzeichnet, dass**
die Temperaturänderungseinrichtung (8) dazu angeordnet ist, das Blut in dem venösen Schlauchsegment zyklisch zu erwärmen und/oder abzukühlen; und
die Vorrichtung dazu angeordnet ist, ein Eintreten einer Rezirkulation bei Erfassen einer ersten Größenänderung an der Erfassungseinrichtung (3) zu bestimmen und ein Verschwinden der Rezirkulation bei Erfassen einer zweiten Größenänderung an der Erfassungseinrichtung (3) zu bestimmen, einen ersten Blutfluss bei der ersten Größenänderung und einen zweiten Blutfluss bei der zweiten Größenänderung mathematisch zu einem resultierenden Blutfluss zu kombinieren und die Pumpe (4) dazu anzusteuern, den resultierenden Blutfluss als einen optimalen Blutfluss einzustellen.

2. Vorrichtung nach Anspruch 1, bei der die Temperaturänderungseinrichtung (8) stromab eines Blutausgangs eines Dialysators (6) angeordnet ist.

3. Vorrichtung nach einem der vorangehenden Ansprüche, bei der eine Temperaturerfassungseinrichtung (9) vorgesehen und dazu angeordnet ist, die Temperatur des Bluts an dem oder in dem venösen Schlauchsegment zu erfassen.

4. Vorrichtung nach einem der vorangehenden Ansprüche, bei der die Temperaturänderungseinrichtung (8) ein Peltier-Element ist.

5. Vorrichtung nach einem der vorangehenden Ansprüche, bei der die physikalische Größe, die sich mit der Temperaturänderung durch die Temperaturänderungseinrichtung (8) einhergehend ändert, eine einer arteriellen Bluttemperatur zugeordnete Temperatur des Bluts, ein Druck oder eine Kraft ist.

6. Vorrichtung nach einem der vorangehenden Ansprüche, die dazu angeordnet ist, durch das zyklische Erwärmen und Abkühlen des Bluts in dem venösen Schlauchabschnitt einen Temperatursignalverlauf an zumindest einer Temperaturerfassungseinrichtung (3) zu erzeugen, wobei Amplituden der und/oder Flächen unter dem Temperatursignalverlauf zur quasi-kontinuierlichen Bestimmung einer Rezirkulation auswertbar sind.

7. Vorrichtung nach einem der vorangehenden Ansprüche, bei der eine Rezirkulation durch eine quantitative Maßzahl oder eine qualitative Größe bezüglich medizinischer Relevanz der Rezirkulation bestimmbar ist, wobei die qualitative Größe anhand eines prozentualen Schwellenwerts "Rezirkulation gleich oder größer als X Prozent" mit X als einer zahlenmäßigen Prozentangabe ausgebbar ist.

## Claims

1. Device for recirculation measurement by means of thermodilution during extracorporeal blood treatment, having:
a temperature change device (8), which is arranged to heat and/or to cool blood in a venous line segment of the blood line system for an at least almost continuous recirculation measurement; and
a detection device (3) which is configured to measure a change of a physical quantity in an arterial line segment of the blood line system, said change resulting from the heating and/or cooling of the blood, wherein
the detection device (3) is arranged to detect a first physical quantity in a case free of recirculation in which no recirculation from venous blood into arterial blood occurs during the extracorporeal blood treatment, and to detect a physical quantity changed by the cooling and/or heating of the blood in the venous line segment by the temperature change device (8) as a second physical quantity in the blood line system in a case involving recirculation in which a recirculation of venous blood into arterial blood occurs during the extracorporeal blood treatment;
a pump (4) which is arranged to pump blood in a blood line system of an extracorporeal blood circuit, wherein the pump (4) is able to be controlled depending on a quantity change detected at the detection device (3) to set an optimal blood flow for a dialysis treatment, **characterised in that**
the temperature change device (8) is arranged to heat and/or to cool the blood cyclically in the venous line segment; and
the device is arranged to determine an occurrence of a recirculation in the case of detection of a first quantity change at the detection device (3) and to determine a disappearance of the recirculation in the case of detection of a second quantity change at the detection device (3), to combine mathematically a first blood flow in the case of the first quantity change and a second blood flow in the case of the second quantity change into a resulting blood flow and to control the pump (4) to set the resulting blood flow as an optimal blood flow.

2. Device according to claim 1, in which the temperature change device (8) is arranged downstream of a blood outlet of a dialysis machine (6).

3. Device according to any one of the preceding claims, in which a temperature detection device (9) is provided and arranged to detect the temperature of the blood at the or in the venous line segment.

4. Device according to any one of the preceding claims, in which the temperature change device (8) is a Peltier element.

5. Device according to any one of the preceding claims, in which the physical quantity which changes with the temperature change due to the temperature change device (8) is a temperature of the blood allocated to an arterial blood temperature, a pressure or a force.

6. Device according to any one of the preceding claims, which is arranged to generate a temperature signal path at at least one temperature detection device (3) due to the cyclical heating and cooling of the blood in the venous line section, wherein amplitudes of and/or planes below the temperature signal path are able to be evaluated for the almost continuous determination of a recirculation.

7. Device according to any one of the preceding claims, in which a recirculation is able to be determined by a quantitative measured value or a qualitative quantity with regards to medical relevance of the recirculation, wherein the qualitative quantity is able to be output by means of a percentage threshold value "recirculation the same as or greater than X percent" where X is a numerical percentage value.

## Revendications

1. Dispositif servant à la mesure de recirculation au moyen d'une thermodilution lors d'un traitement extracorporel du sang, avec :
un système de modification de température (8), qui est disposé pour réchauffer et/ou refroidir, pour une mesure de recirculation au moins quasi continue, du sang dans un segment de tube flexible veineux du système de tubes flexibles pour le sang ; et
un système de détection (3), qui est configuré pour mesurer une modification, résultant du réchauffage et/ou du refroidissement du sang, d'une grandeur physique dans un segment de tube flexible artériel du système de tubes flexibles pour le sang, dans lequel
le système de détection (3) est disposé pour détecter dans un cas sans recirculation, dans lequel, lors du traitement extracorporel du sang, aucune recirculation de sang veineux dans du sang artériel n'a lieu, une première grandeur physique et pour détecter dans le cas d'un cas inhérent à la recirculation, dans lequel, lors du traitement extracorporel du sang, une recirculation de sang veineux dans du sang artériel a lieu, une grandeur physique modifiée par le refroidissement et/ou le réchauffage du sang dans le segment de tube flexible veineux par le système de modification de température (8) en tant qu'une deuxième grandeur physique dans le système de tubes flexibles pour le sang ;
une pompe (4), qui est disposée pour pomper du sang dans un système de tubes flexibles pour le sang d'un circuit sanguin extracorporel, dans lequel la pompe (4) peut être pilotée en fonction d'une modification de grandeur détectée au niveau du système de détection (3) pour régler un débit de sang optimal pour un traitement par dialyse,
**caractérisé en ce que**
le système de modification de température (8) est disposé pour réchauffer et/ou refroidir de manière cyclique le sang dans le segment de tube flexible veineux ; et
le dispositif est disposé pour définir une survenue d'une recirculation lors de la détection d'une première modification de grandeur au niveau du système de détection (3) et pour définir une disparition de la recirculation lors de la détection d'une deuxième modification de grandeur au niveau du système de détection (3), pour combiner un premier débit de sang lors de la première modification de grandeur et un deuxième débit de sang lors de la deuxième modification de grandeur de manière mathématique pour obtenir un débit de sang en résultant et pour piloter la pompe (4) pour régler le débit de sang résultant en tant qu'un débit de sang optimal.

2. Dispositif selon la revendication 1, où le système de modification de température (8) est disposé en aval d'une sortie de sang d'un dialyseur (6).

3. Dispositif selon l'une quelconque des revendications précédentes, où un système de détection de température (9) est prévu et disposé pour détecter la température du sang au niveau du ou dans le segment de tube flexible veineux.

4. Dispositif selon l'une quelconque des revendications précédentes, où le système de modification de température (8) est un élément Peltier.

5. Dispositif selon l'une quelconque des revendications précédentes, où la grandeur physique, qui varie de pair avec la modification de température par le système de modification de température (8), est une température, associée à une température du sang artériel, du sang, une pression ou une force.

6. Dispositif selon l'une quelconque des revendications précédentes, qui est disposé pour générer, par le réchauffage et le refroidissement cycliques du sang dans la section de tube flexible veineuse, un profil de signal de température au niveau d'au moins un système de détection de température (3), dans lequel des amplitudes et/ou des surfaces peuvent être évaluées selon le profil de signal de température pour définir de manière quasi continue une recirculation.

7. Dispositif selon l'une quelconque des revendications précédentes, où une recirculation peut être définie par une valeur de mesure quantitative ou une grandeur qualitative en termes de pertinence médicale de la recirculation, dans lequel la grandeur qualitative peut être éditée à l'aide d'une valeur de seuil exprimée en pourcentage « recirculation égale ou supérieure à X pour cent » avec X en tant qu'une indication de pourcentage chiffrée.
